# EUROPEAN PATENT APPLICATION

(11) **EP 2 899 207 A1**
(43) Date of publication of application: **29.07.2015**
(21) Application number: 14000305.4
(22) Date of filing: 28.01.2014
(51) Int. Cl.: C07K 14/81, C07K 14/005, C12Q 1/02, C12Q 1/37

(54) **New method for testing HCV protease inhibition**

(71) Applicant: Amikana.Biologics, 13005 Marseille (FR)
(72) Inventor: Gluschankof, Pablo, 13001 Marseille (FR); Perrin-East, Christèle, 13009 Marseille (FR)
(74) Representative: Barbot, Willy

(57) **Abstract**

The present invention relates to a vector coding for an antitoxin comprising a nucleic acid sequence under the control of a promoter, wherein (i) said nucleic acid sequence encodes a pro-antitoxin comprising the sequence of a cleavage site of a HCV protease, and (ii) said pro-antitoxin has to be cleaved by said HCV protease to be activated; a cell transformed by such a vector, a method using such transforming cell for testing the ability of a compound to inhibit a HCV protease; and a kit for testing the ability of a compound to inhibit HCV protease.

## Description

### Field of the Invention

The present invention is directed to a new vector for testing the ability of a compound to inhibit HCV protease; a cell transformed by said vector; a method of using such a vector.

### Background of the invention

Infection by hepatitis C virus ("HCV") is a compelling human medical problem. HCV is recognized as the causative agent for most cases of non-A, non-B hepatitis, with an estimated human sero-prevalence of 3% globally.

Upon first exposure to HCV only about 20% of infected individuals develop acute clinical hepatitis while others appear to resolve the infection spontaneously. In almost 70% of instances, however, the virus establishes a chronic infection that persists for decades. This usually results in recurrent and progressively worsening liver inflammation, which often leads to more severe disease states such as cirrhosis and hepatocellular carcinoma. Unfortunately, there are no broadly effective treatments for the debilitating progression of chronic HCV.

The HCV genome encodes a polyprotein of 3010-3033 amino acids (CHOO et al., Proc. Natl. Acad. Sci. U.S.A., vol.88, p:2451-2455, 1991; KATO et al., Proc. Natl. Acad. Sci. U.S.A, vol.87, p:9524-9528, 1990; TAKAMIZAWA et al., J. Virol., vol.65, p:1105-1113, 1991). The HCV nonstructural (NS) proteins are presumed to provide the essential catalytic machinery for viral replication, which are derived by proteolytic cleavage of the polyprotein (BARTENSCHLAGER et al., J. Virol., vol.67, p:3835-3844, 1993; GRAKOUI et al., J. Virol., vol.67, p:2832-2843, 1993; GRAKOUI et al., J. Virol., vol.67, p:1385-1395, 1993; TOMEI et al., J. Virol., vol.67, p:4017-4026, 1993).

The HCV NS protein 2 (NS2) and HCV NS protein 3 (NS3) present, a cysteine and a serine protease activity respectively, which are implicated in the maturation of the viral polyprotein encoded by the genome.

Thus, both NS2 and NS3 proteins are considered as essentials for viral replication and infectivity. Moreover, the HCV NS2 protein acts to coordinate virus assembly and the HCV NS3 is known to be a multifunctional protein harboring also a helicase and nucleoside triphosphate activities.

The protease activity of HCV NS3 serine protease and its NS4A co-factor, also known as the NS3-4A complex, is essential for viral replication since the substitutions of the catalytic triad resulted in loss of infectivity in chimpanzees. The first 181 amino acids of NS3 (residues 1027-1207 of the viral polyprotein) have been shown to contain the serine protease domain of NS3 that processes all four downstream sites of the HCV polyprotein (LIN et al., J. Virol., vol.68, p:8147-8157, 1994). The HCV NS3 serine protease and its associated cofactor, NS4A, help process the viral non-structural protein region into individual non-structural proteins, including all of the viral enzymes. This processing appears to be analogous to that carried out by the human immunodeficiency virus aspartyl protease, which is also involved in processing of viral proteins.

HCV protease inhibitors, which inhibit viral protein processing, are potent antiviral agents in human, indicating that interrupting this stage of the viral life cycle results in therapeutically active agents. Consequently, it is an attractive target for drug discovery.

Several potential HCV protease inhibitors, which are mainly NS3 inhibitors, have been described in the prior art. See for example International patent applications Nos. WO 2012/049688, WO 2012/040167, WO 2011/049908, WO 2011/005646, WO 2010/132163, WO 2010/017432, WO 2009/076173, WO 2009/085978, WO 2009/076166, WO 2009/053828, WO 2009/152051, WO 2009/134987, WO 2009/120783, WO 2009/117594, WO 2009/094443, WO 2009/094438, WO 2009/085978, WO 2009/079353, WO 2009/079352, WO 2009/073719, WO 2009/073713, WO 2009/070692, WO 2009/070689, WO 2009/064975, WO 2009/064955, WO 2008/134398, WO 2008/134397, WO 2008/134395, WO 2008/106058, WO 2008/02200, WO 2008/021960, WO 2008/021956, WO 2008/021871, WO 2007/146695, WO 2007/089618, WO 2007/143694, WO 2006/130554, WO 2006/086381, WO 2006/039488, WO 2005/113581, WO 2005/077969, WO 2005/073216, WO 2005/037860, WO 2004/113365, WO 2004/093798, WO 2004/092161, WO 2004/072243, WO 03/087092, WO 03/066103, WO 03/035060, WO 03/006490, WO 02/18369, WO 02/08244, WO 00/09558, WO 00/09543, WO 99/64442, WO 99/07733, WO 99/07734, WO 99/50230,WO 98/46630, WO 98/17679 and WO 97/43310.

Preferably, said NS3 inhibitors are selected in the group comprising boceprevir, telaprevir, asunaprevir, faldaprevir, ABT-450, simeprevir, danoprevir, GS-9451, and MK-5172.

It is not known however whether all these compounds would have the appropriate profiles to be acceptable drugs. Furthermore, most, if not all of these inhibitors were discovered using one genotype, mostly the genotype 1 (1a or 1b) NS3-4A serine protease as the target. However, there are a variety of genotypes of HCV, and a variety of subtypes within each genotype. For example, at present it is known that there are eleven (numbered 1 through 11) main genotypes of HCV, although others have classified the genotypes as 6 main genotypes. Each of these genotypes is further subdivided into subtypes (1a - 1c; 2a-2c; 3a-3b; 4a-4e; 5a; 6a; 7a-7b; 8a-8b; 9a; 10a; and 11a).

The prevalence of the subtypes varies globally as listed in the following Table I.

**Table I**

| HCV genotype | Geographic main location |
|---|---|
| 1a | Found mostly in North and South America; common in Australia |
| 1b | Found mostly in Europe and Asia |
| 2a | Most common genotype 2 in Japan and China |
| 2b | Most common genotype 2 in US and Northern Europe |
| 2c | Most common genotype 2 in Western and Southern Europe |
| 3a | Highly prevalent in Australia and South Asia |
| 4a | Highly prevalent in Egypt |
| 4c | Highly prevalent in Central Africa |
| 5a | Highly prevalent in South Africa |
| 6a | Restricted to Hong Kong, Macau and Vietnam |
| 7a & 7b | Common in Thailand |
| 8a, 8b & 8c | Prevalent in Vietnam |
| 10a and 11a | Found in Indonesia |

Now, the HCV genotype or subtype can determine the responsiveness of the patient to therapy. In fact, it is generally accepted that genotype 2 HCV and genotype 3 HCV virus-infected patients respond to conventional therapy to a different degree than those patient infected with genotype 1 HCV. Thus, while a number of HCV protease inhibitors have been designed/discovered against genotype 1 HCV protease, it is not clear whether these inhibitors will effectively inhibit the HCV NS3-4A serine proteases from other genotypes, such as for example genotype 2 HCV and genotype 3 HCV.

Therefore, the current understanding of HCV has not led to any satisfactory anti-HCV agents or treatments.

Thus, there is a need for more effective anti-HCV therapies, particularly new compounds that inhibit HCV NS3 protease. Such compounds may be useful as antiviral agents, particularly as anti-HCV agents.

There is also a need for compounds that inhibit various genotypes of the HCV serine protease

### Summary of the Invention

The present invention is directed to a new method enabling to simply test the activity of a compound against a HCV protease. This method permits to screen for new potential HCV protease inhibitors and also to adapt the protocol of a patient infected by HCV so as to obtain the best therapeutic response.

Accordingly, a first object of the invention is directed to a vector coding for an antitoxin comprising a nucleic acid sequence under the control of a promoter, wherein (i) said nucleic acid sequence encodes a pro-antitoxin comprising the sequence of the cleavage site of a HCV protease, and (ii) said pro-antitoxin has to be cleaved by said HCV protease to be activated.

According to a preferred embodiment, the antitoxin is directed against the K28 toxin activity, which is encoded by the K28 pre-pro-toxin (SEQ id n°1, Accession number 2205370A). The K28 pre-pro-toxin polypeptide is processed by the cell to a biologically active α/β heterodimeric protein that kills sensitive yeast cells via a two-step receptor mediated infecting mechanism where the β sub-unit acts as a docking element to the cell surface receptor, allowing the α sub-unit to enter the cell nucleus and to perform its toxic activity by inhibiting DNA replication (BOSTIAN et al., Cell, vol.36, p:741-751, 1984, SCHMITT & TIPPER, Virology, vol.213, p:341-351, 1995). Moreover, when directly expressed into the cell, the K28 pre-pro alpha subunit (SEQ id n°2) induces the death of the cell. Accordingly, the toxin is preferably the K28 pre-pro alpha subunit (SEQ id n°2).

Now, it was found that the α sub-unit alone (SEQ id n°3, wherein the first amino acid has been substituted by a methionine), has a mild anti-toxin activity toward K28 infection when expressed in the infected cells.

According to still another preferred embodiment, the antitoxin is a C terminal extended α sub-unit polypeptide, which C-terminal extremity consists of the 25 first amino-acids of the gamma sub-unit SEQ id n°109.

According to still another preferred embodiment, the pro-antitoxin is a polypeptide, which N-terminal extremity comprises a derivative of the sequence SEQ id n°109, said derivative consisting in the insertion of a HCV cleavage site between the position 100 and 126 of SEQ id n°109, preferably said derivative is SEQ id n°110.

According to another preferred embodiment, the HCV protease is NS3-4A (For example SEQ id n°115) and the NS3-4A protease site is selected in the group comprising SEQ id n°4 to 108.

Advantageously, the pro-antitoxin is a polypeptide consisting in the sequence SEQ id n°110.

Another object of the invention is directed to a cell, preferably a yeast cell, transformed with the vector as defined previously.

Now, such cell may be further transformed by a vector coding for the toxin K28 under the control of an inducible promoter.

This cell may be also transformed by a vector coding for the HCV protease activating the pro-antitoxin. Such cell may be obtained by its co-transformation with:

i) a DNA fragment, obtained by amplifying at least one sequence coding for a HCV protease, and

ii) a vector enabling the expression of said HCV protease in the cell after a correct recombination step with the DNA fragment.

In a preferred embodiment, this HCV protease is NS3-4A.

Another object of the invention is directed to a method for screening a compound with the ability to inhibit HCV protease comprising the steps of:

i) contacting, with the compound, the cell transformed with:

a) a vector coding for an antitoxin comprising a nucleic acid sequence under the control of a promoter, wherein (i) said nucleic acid sequence encodes a pro-antitoxin comprising the sequence of the cleavage site of a HCV protease, and (ii) said pro-antitoxin has to be cleaved by said HCV protease to be activated; and

b) a vector coding for the HCV protease activating the pro-antitoxin.

ii) culturing the cell in a selective media so as to contact the cell with the toxin, and

iii) detecting inhibition of growth of the cell, a compound that inhibits growth of the cell being a compound that inhibits HCV protease.

In a preferred embodiment, the method of the invention is for determining the IC₅₀ of the compound for a HCV protease, wherein the successive steps i), ii) and iii) are repeated with different concentration of the compound.

In still another preferred embodiment, the method of the invention is for determining the ability of the compound to inhibit the HCV proteases expressed by different isolates of HCV viruses, wherein the successive steps i), ii) and iii) are repeated with distinct transformed cells, each transformed cell expressing the HCV protease of one isolate of HCV virus.

In a further preferred embodiment, the method of the invention is for determining an efficient therapeutic regimen that can be used for treating a subject suffering from a HCV infection, wherein the successive steps i), ii) and iii) are repeated with each compound to be tested with:

a) said compound being selected in the group comprising inhibitors of a HCV protease, and

b) said transformed cell corresponds to a pool of transformed cells, which pool of cells has been transformed by:

1) a vector coding for an antitoxin comprising a nucleic acid sequence under the control of a promoter, wherein said nucleic acid sequence encodes a pro-antitoxin comprising the sequence of the cleavage site of the HCV protease, and said pro-antitoxin has to be cleaved by said HCV protease to be activated; and

2) a pool of vectors coding for the pool of HCV proteases expressed by the HCV viruses infecting said subject.

Finally, an object of the invention is directed to a kit for screening a compound having the ability to inhibit a HCV protease, wherein said kit comprises:

i) a cell transformed by a vector coding for an antitoxin comprising a nucleic acid sequence under the control of a promoter, wherein said nucleic acid sequence encodes a pro-antitoxin comprising the sequence of the cleavage site of a HCV protease, and said pro-antitoxin has to be cleaved by said HCV protease to be activated;

ii) a set of primers for amplifying a DNA fragment comprising at least one sequence coding for a HCV protease; and

iii) a vector enabling the cloning of said DNA fragment and the expression of said HCV protease in the cell after its transformation.

### Detailed Description of the invention

The invention aims to provide a new system for testing the inhibition of a HCV protease by a compound.

In a first object, the invention relates to a vector coding for an antitoxin comprising a nucleic acid sequence under the control of a promoter, wherein (i) said nucleic acid sequence encodes a pro-antitoxin comprising the sequence of the cleavage site of a HCV protease, and (ii) said pro-antitoxin has to be cleaved by said HCV protease to be activated.

As used herein an antitoxin refers to a polypeptide neutralizing the activity of a toxin.

As used herein, a "toxin" refers to a poisonous substance, said substance inducing cell death.

Examples of toxins comprise cyanotoxins, microbial toxins, mycotoxins, insect toxins, and virus toxins. Preferably, said toxin is a cytotoxin.

Still preferably, said toxin is a yeast killer toxin such as K1, K2 or K28, and most preferably, said toxin is K28.

As used herein, an "antitoxin" refers to a polypeptide having the ability of neutralizing the toxin. When neutralized by the antitoxin, the toxin is no more poisonous for the cells.

As used herein, the term "pro-antitoxin" refers to a polypeptide that is unable to neutralize the toxin, said pro-antitoxin needing to be activated by a HCV protease so as to have said neutralizing activity.

Most preferably, the pro-antitoxin is a polypeptide, which N-terminal extremity comprises or consists in a derivative of the sequence SEQ id n° 109, said derivative consisting in the insertion of a HCV cleavage site between the position 100 and 126 of SEQ id n°109, preferably said derivative is SEQ id n°110.

Now, this HCV protease is preferably NS3-4A and the NS3-4A protease site is selected in the group comprising SEQ id n°4 to 108.

As an example of such an antitoxin, one can cite the polypeptide consisting in the sequence SEQ id n° 110.

As used herein, the term "promoter" refers to a DNA sequence capable of directing transcription (thus expression) of a gene or a coding sequence which is operably linked to said promoter.

Generally, the expression "under the control of" means that the transcriptional regulatory nucleic acid is positioned relative to any coding sequence in such a manner that transcription is initiated. As used herein, this will mean that the promoter is positioned 5' to the coding region, at a distance allowing the expression of said coding region. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental conditions.

There are different kinds of promoters. A promoter as used herein may be an inducible or regulated promoter (its activity is up or down regulated by the presence or absence of biotic or abiotic factors) or a constitutive promoter (it leads to a gene expression in most cell types at most times). A promoter used according to the invention may be a strong promoter (leading to a high gene expression) or weak promoter (leading to a low gene expression) (MAYA et al., Biotechnol. Lett., vol.30, p:979-987, 2008).

Promoters used according to the invention are functional in yeast cells. Examples of promoters functional in yeast that could be used in the present invention comprise, but are not limited to: the GAL1 promoter (SEQ ID n°111), the ADH1 promoter (SEQ ID n°112) and the strong GPD (Glyceraldehyde 3P DH) promoter (SEQ ID n°113). (MAYA et al., Biotechnol. Lett., vol.30, p:979-987, 2008).

The term "vector" means a vehicle by which a nucleic acid sequence can be introduced into a host cell, so as to transform the host and promote expression (e.g. transcription and translation) of the introduced nucleic acid sequence.

Typically, such a vector is selected in the group comprising plasmid, cosmid, episome, artificial chromosome, phage or a viral vector.

Preferably, said vector is a plasmid.

Such vector comprises necessary elements well known by one skilled in the art, such as an origin of replication, preferably an origin of replication functional in yeast such as ars 1, centromere ori, and 2µ ori. Such vector may also comprise other regulatory elements, such as an enhancer, a terminator, a polyadenylation region and the like, to cause or direct expression of said antitoxin after transformation of a cell.

This vector may also comprise a selectable marker.

Selectable markers functional in yeast are survival genes including ALG7, ADE2, CUP1, HIS3, LEU2, or TRP1.

Another object of the invention refers to a cell transformed with the vector as defined previously.

The term "transformation" or "transforming" refers to the transfer of a DNA fragment, a plasmid or a vector into a host organism. Host organisms containing such DNA fragment, plasmid or vector are called "recombinant" or "transformed" organisms.

In the method of the invention, the transformed cell is preferably a yeast cell, such as *Saccharomyces cerevisiae, Candida albicans, Candida maltosa, Hansenula polymorpha, Kluyveromyces fragilis, Kluyveromyces lactis, Pichia guillerimondii, Pichia pastoris, Schizosaccharomyces pombe*, and *Yarrowia lipolytica.* Preferably, the transformed yeast cell is from *Saccharomyces cerevisiae* species.

According to a preferred embodiment, the cell of the invention is further transformed by a vector coding for a toxin under the control of an inducible promoter.

Preferably, said toxin is the K28 pre-pro- α subunit SEQ id n°2.

The used inducible promoter is functional in the transformed cell of the invention, and preferably in yeast cells. Examples of inducible promoters functional in yeast that could be used in the present invention comprise, but are not limited to: GAL1/10 promoter (GUARENTE et al., PROC.NATL.ACAD.SCI.,vol 79, p:7410-7414, 1982) (SEQ id n°111), Tetracycline-Regulated (tetR) promoter (GARI et al., Yeast, vol 13, p:837-848, 1997) (SEQ id n°114).

According to still another preferred embodiment, the cell of the invention is further transformed by a vector coding for the HCV protease activating the pro-antitoxin.

Preferably, said HCV protease is NS3-4A protease. As an example of NS-34A protease sequence, one can cite SEQ id n° 115.

Still, preferably, said HCV protease is under the control of an inducible or constitutive promoter.

For obtaining this particular cell, said cell can be co-transformed by i) a DNA fragment, obtained by amplifying at least one sequence coding for a HCV protease, and ii) a vector enabling the expression of said HCV protease in the cell after a correct recombination step with the DNA fragment.

Preferably, said HCV protease is NS3-4A and is amplified by example by the pair of primers SEQ id n°127 and SEQ id n° 128, or SEQ id n°129 and SEQ id n° 130.

In order to induce the insertion of the amplified DNA fragment coding for NS3-4 HCV protease in any chosen vector through homologous recombination, the sequence of the primers used for amplification, namely SEQ id n°127 and SEQ id n°128, or SEQ id n°129 and SEQ id n°130, include at their 5' end a sequence of 20 bp homologous to the vector sequence where the viral encoded DNA have to be inserted.

The vector enabling the expression of said HCV protease in the cell after a correct recombination step with the DNA fragment can be simply identified by the skilled person. As an example, such vector is selected in the group comprising pRS316, and p413CYC1, p413TEF, p413ADH, p423GPD, p423CYC1, p423TEF, p423ADH, p423GPD, p414CYC1, p414TEF, p414ADH, p414GPD, p424CYC1, p424TEF, p424ADH, p424GPD, p415CYC1, p415TEF, p415ADH, p415GPD, p425CYC1, p425TEF, p425ADH, p425GPD, p416CYC1, p416TEF, p416ADH, p416GPD, p426CYC1, p426TEF, p426ADH, p426GPD, and preferably said vector is p415ADH.

Another object of the invention is directed to a method for screening a compound with the ability to inhibit HCV protease comprising the steps of:

i) contacting a cell transformed by:

(a) a vector coding for an antitoxin comprising a nucleic acid sequence under the control of a promoter, wherein said nucleic acid sequence encodes a pro-antitoxin comprising the sequence of the cleavage site of a HCV protease, and said pro-antitoxin has to be cleaved by said HCV protease to be activated; and

(b) a vector coding for the HCV protease activating the pro-antitoxin;

ii) culturing the cell in a selective media so as to contact the cell with the toxin, and

iii) detecting inhibition of growth of the cell, a compound that inhibits growth of the cell being a compound that inhibits HCV protease.

The transformed cell is preferably a transformed yeast, and most preferably *Saccharomyces cerevisae*.

As used herein, a selective media corresponds to a culture media enabling the growth of the cells, which culture media has been modified with either the addition of the toxin (if not encoded by a vector) or with the addition of the inducer of the inducible promoter controlling the expression of the toxin (if encoded by a vector). The expression "addition of the inducer" is related to both addition a particular element -i.e. galactose- and to deletion of an element -i.e. tetracycline- resulting in the specific activation of the inducible promoter activating the toxin.

In a first preferred embodiment, the toxin is the K28 pre-pro- α subunit (SEQ id n°2) and the cell of step (i) was further transformed by a vector coding for the K28 pre-pro-α subunit under the control of an inducible promoter.

Then, the antitoxin is directed against the pre-pro-α subunit of K28.

The pro-antitoxin is a polypeptide, which N-terminal extremity comprises or consists in a derivative of the sequence SEQ id n°109, said derivative consisting in the insertion of a HCV cleavage site between the position 100 and 126 of SEQ id n°109, preferably said derivative is SEQ id n°110.

According to another preferred embodiment, the HCV protease is NS3-4A and the NS3-4A protease site is selected in the group comprising SEQ id n° 4 to 108.

According to still another preferred embodiment, the pro-antitoxin is a polypeptide consisting in the sequence SEQ id n°110.

The tested compound may be selected from a group comprising the molecules of a library, chemical molecules, natural molecules and molecules extracted from plants. Now, the compound may be also known to have a HCV protease inhibition activity. In this hypothesis, the method of the invention may have different purposes 1) determining the IC₅₀ of the compound, 2) determining the scope of the protease inhibition activity on different HCV genotypes and/or subtypes, and/or 3) determining an efficient therapeutic regimen.

In another preferred embodiment, the method of the invention is for determining the IC₅₀ of the compound for a HCV protease, wherein the successive steps i), ii) and iii) are repeated with different concentration of the compound.

In still another preferred embodiment, the method of the invention is for determining the ability of the compound to inhibit the HCV proteases expressed by different isolates of HCV viruses, wherein the successive steps i), ii) and iii) are repeated with distinct transformed cells, each transformed cell expressing the HCV protease of one isolate of HCV virus.

In a further preferred embodiment, the method of the invention is for determining an efficient therapeutic regimen that can be used for treating a subject suffering from a HCV infection, wherein the successive steps i), ii) and iii) are repeated with each compound to be tested with:

a) said compound being selected in the group comprising inhibitors from a HCV protease, and

b) said transformed cell corresponds to a pool of transformed cells, which pool of cells has been transformed by:

1) a vector coding for an antitoxin comprising a nucleic acid sequence under the control of a promoter, wherein said nucleic acid sequence encodes a pro-antitoxin comprising the sequence of a cleavage site of such a HCV protease, and said pro-antitoxin has to be cleaved by said HCV protease to be activated; and

2) a pool of vectors coding for the pool of HCV proteases expressed by the HCV viruses infecting said subject.

Such a pool of vectors can be simply obtained by inserting the DNA fragments amplified by RT-PCR out of total viral RNA from infected blood plasma individuals into the said expressing vectors.

Still another object of the invention is directed to a kit for screening a compound with the ability to inhibit HCV protease, wherein said kit comprises:

i) a cell transformed by a vector coding for an antitoxin comprising a nucleic acid sequence under the control of a promoter, wherein said nucleic acid sequence encodes a pro-antitoxin comprising the sequence of the cleavage site of a HCV protease, and said pro-antitoxin has to be cleaved by said HCV protease to be activated

ii) a set of primers for amplifying a DNA fragment comprising at least one sequence coding for a HCV protease; and

iii) a vector enabling the cloning of said DNA fragment and the expression of said HCV protease in the cell after its transformation.

Preferably, said HCV protease is NS3-4A and is amplified by example by the pair of primers SEQ id n°127 and SEQ id n°128, or SEQ id n°129 and SEQ id n°130.

In a preferred embodiment, said kit comprises a selective media enabling to contact the transformed cell with the toxin.

In another preferred embodiment, the cell was also transformed by a vector coding for the toxin under the control of an inducible promoter.

Preferably, the toxin is the K28 pre-pro- α subunit (SEQ id n°2).

Preferably, the pro-antitoxin and the antitoxin are as described previously.

In the following examples, the invention is described in more detail with reference to methods. Yet, no limitation of the invention is intended by the details of the examples. Rather, the invention pertains to any embodiment which comprises details which are not explicitly mentioned in the examples herein, but which the skilled person finds without undue effort.

### Examples

### 1) Design of a toxin/antitoxin system

### 1/ K28 minimal sequence:

The integral alpha subunit sequence (SEQ id n°3, amino acid 50 to amino acid 149 of the M28 sequence) or its N-terminal extended prepro-form (SEQ id n°2, amino acid 1 to amino acid 149) were amplified by PCR from the plasmid pDT-M28 containing the complete cDNA copy of M28 ds RNA with primers 5'K28A and 3'K28sac for the K28Alpha subunit coding sequence and primers 5'K28ppA and 3'K28sac for the K28-pre-pro-alpha (K28ppAlpha) subunit coding sequence.

5'primer K28A gaatgtgggtctagaatggacttcagtgctgctacttgc (SEQ id n°116)

5'primer K28ppA: tctagactcgaggaattcatggagagcgtttcctcattattt (SEQ id n°117)

3'primerK28sac: gagctcttaacgcgattgtatattctctgc (SEQ id n°118)

These primers allowed both amplification of DNA coding sequences being framed by codons for transcriptional initiation -i.e. ATG-, and for transcriptional termination -i.e. TAA-, and a unique cleavage site for restriction enzyme was added at each DNA end: XbaI at the 5' end and SacI at the 3' end.

The vector pRS-Ga11/10, containing the Galactose inducible promoter Ga11/10 in position 5' to the cloning site of the gene to be expressed and the PCR amplified DNA fragments were digested with XbaI and SacI restriction enzymes. Each DNA digested fragment was further ligated to the digested vector through overnight incubation withT4 DNA ligase (from GIBCO)The obtained vectors, pRS316Ga11/10-K28Alpha and pRS316Ga11/10-K28ppAlpha, after amplification through bacteria transformation, were introduced in yeast cells through transformation procedure. In this example the yeast strains used were either BY4742 or W303 from *Saccharomyces cerevisiae.*

K28Alpha subunit dependent cellular toxicity was tested as follows: approximately 10⁵ cells expressing K28Alpha subunit in Galactose containing media were either plated on a galactose containing solid medium or seeded in 1ml of galactose containing liquid minimal media and incubated at 30°C for 48hrs. Media turbidity, accounting for cell growth, was determined by absorbance measurement at 600nm when the test was performed in liquid media. If the test was performed in solid medium living cells were detected by eye on cell colony formation.

It was observed that the K28Alpha subunit N-terminally extended with the prepro sequence (SEQ id n°2) was toxic to the cell, whereas expression of the K28Alpha subunit alone (SEQ id n°3) did not induce any cell death.

### 2/ anti-toxin minimal sequence

### In order to determine the minimal sequence presenting the anti-toxin activity, we co-expressed different deleted forms of the K28 sequence with the pre-pro-α toxic subunit (SEQ id n°2). K28 derived sequences were produced by PCR amplification of different regions of the K28 coding gene with primers 5'alpha and 3'M28, or 3'M28-100 or primers 5'beta and 3'M28, or 3'M28-100.

5'alpha: Gaatgtgggtctagaatggacttcagtgctgctacttgc (SEQ id n°119)

3'M28-100: ttaagctcgtttctgcaagcctgtaga (SEQ id n°120)

3'M28-170: Gctcttagaatacgtcttccaaattaatcgc (SEQ id n°121)

5'beta: Gaatgtgggtctagaatgcagcatctagctgaagctaacggt (SEQ id n°122)

3'M28: gtctggactattagcgtagctcatcgtggcacct (SEQ id n°123)

Yeast cells BY4742 harboring the pRS316Ga11/10-ppAlpha plasmid were transformed with plasmids generated by introducing the created mutations in the expression vectors p415adh or p413gpd.

As K28 pre-pro-Alpha subunit is lethal to the expressing cell, anti-toxin activity of K28 derived proteins was determined as cell growth in Galactose of cells co-expressing a particular K28 derived sequence and K28 pre-pro-Alpha subunit.

Anti-toxin activity was tested as follows: approximately 10⁵ cells expressing K28 pre-pro-Alpha subunit and a specific K28 derived protein in Galactose containing media were either plated on a galactose containing solid medium or seeded in 1ml of galactose containing liquid minimal media and incubated at 30°C for 48hrs. Media turbidity, accounting for cell growth, was determined by absorbance measurement at 600nm when the test was performed in liquid media. If the test was performed in solid medium living cells were detected by eye on cell colony formation.

We have found that expression of a sequence coding for the K28 Alpha subunit C-terminally extended by the first 25 amino acids of the gamma subunit (K28Alpha-25gamma) was able to inhibit the toxic activity of K28Alpha whereas expression of the fully gamma subunit extended form had shown no anti toxic activity.

3/Analysis of HCV NS3 proteolytic activity in yeast cells: Creation of a testing biological system based on a pro-anti-toxin/toxin tandem molecules.

The developed rational for HCV NS3 proteolytic enzyme activity determination is as follows: if K28Alpha-25gamma (SEQ id n°109) has anti toxin activity where K28Alpha-gamma has not, by introducing an HCV NS3 specific cleavage site at the 25^{th} amino acid of the gamma subunit in K28Alpha-gamma, protease action will liberate the anti-toxin activity. When this occurs in a yeast cell expressing K28 pre-pro-Alpha subunit, cell growth will account for HCV NS3 enzyme activity.

In order not to perturb, as much as possible, the amino acid composition of the gamma subunit sequence we first looked for, within the amino acid sequence of the gamma subunit, amino acid stretches bearing strong homology with an HCV NS3 recognizable known cleavage sites. This was found in position 167-173

K28Alpha-gamma was then further modified by site directed mutagenesis at positions 167-173 of the gamma subunit sequence using the following primer:

K28Alpha-gammaNS3 5'primer: ***Acccataccatggcggcggatctggaagtggtgacc*** (SEQ id n° 124)

K28Alpha-gammaNS3 3'primer: *ggtcaccacttccagatccgccgccatggtatgggt* (SEQ id n°125)

After sequencing analysis to confirm insertion of the cleavage site in the K28Alpha-gamma sequence generating the K28Alpha-gammaNS3 mutated coding gene, the PCR amplified DNA fragment was cloned into the p415ADH vector as previously described.

The expected product of HCV NS3 enzymatic action on K28Alpha-gammaNS3, namely K28Alpha-gamma25S, was also generated by PCR amplification from K28Alpha-gammaNS3 using primers 5'alpha (SEQ id n°119), and 3'M28-170ns3 (ttaggtcaccacttccagatccgccgccatggtatgggt, SEQ id n° 126). Then, said product was cloned into p413gpd expressing vector. BY4742 yeast cells, transformed with the plasmid carrying K28 pre-pro-Alpha subunit gene under the control of galactose inducible promoter, were then transformed with either plasmids.

K28Alpha-gammaNS3 did not present a different anti-toxin profile from the wild type K28Alpha-gamma whereas K28Alpha-gamma25S showed anti-toxin activity. This was tested as follows: approximately 10⁵ cells co-expressing K28 pre-pro-Alpha subunit under the expression control of galactose inducible promoter either with K28Alpha-gammaNS3 or K28Alpha-gamma25S were either plated on a galactose containing solid medium or seeded in 1ml of galactose containing liquid minimal media and incubated at 30°C for 48hrs. Media turbidity, accounting for cell growth, was determined by absorbance measurement at 600nm when the test was performed in liquid media. If the test was performed in solid medium living cells were detected by eye on cell colony formation. It was observed that the presence of K28Alpha-gamma25S inhibited cell death induced in Galactose media by K28 pre-pro-Alpha subunit and K28Alpha-gammaNS3 did not.

**2) Method for screening compounds with the ability to inhibit HCV protease**

HCV NS3 protease was RT-PCR amplified out of blood plasma from untreated HCV infected individual with primers NS3Fwd2 and NS3Rev1.

NS3Fwd2 : ATC ACS TGG GGR GCR GAY AC (SEQ id n°127)

NS3Rev1: AAY TTG CCR TAK GTG GAG TAY GT (SEQ id n°128)

The viral protease encoding DNA fragment was inserted in the cloning site of p415adh-NS4A expression vector creating plasmid p415adhNS3-NS4A. BY4742 yeast strain, harboring K28Alpha-gammaNS3 and K28 pre-pro-Alpha subunit, the late under the control of galactose inducible promoter, was further transformed with the p415adhNS3-NS4A plasmid carrying HCV NS3 and NS4A genes, creating yeast strain YAB010.

10⁵ YAB010 cells are seeded in each single well within a 96 well plate. The compound to be tested for its HCV protease inhibitory activity is added. An incubation is performed for 48 hours at 30°C in 300µl of galactose containing liquid media.

At the end of the incubation cell growth is measured by absorbance at 600nm.

Cell growth accounts for HCV NS3 activity as the viral protease produces, through its action, the anti-toxin out of the pro-anti-toxin protein. Therefore HCV NS3 inhibition correlates to cell death measured as low absorbance at 600nm values.

The best inhibiting compound is the one that, at low concentrations, will not allow the incubated cells, from the presented application, to grow in galactose containing media and will be harmless in glucose media.

### 3) Method for determining the ability of a compound to inhibit HCV protease from different isolates.

HCV NS3 protease was RT-PCR amplified out of purified total circulating RNA from blood plasma from HCV infected individuals with primers NS3Fwd2 (SEQ id n°127) and NS3Rev1 (SEQ id n° 128). The viral protease encoding DNA fragment was inserted in the cloning site of p415adh-NS4A expression vector. BY4742 yeast strain, harboring K28Alpha-gammaNS3 and K28 pre-pro-Alpha subunit, the late under the control of galactose inducible promoter, was further transformed with the plasmid carrying a unique HCV NS3 isolate.

In 11 wells within a 96 well plate 10 different concentrations (one well has no protease inhibitor) of a viral protease inhibiting compound (one concentration per well) are incubated 48 hours at 30°C in 300µl of galactose containing liquid with 10⁵ BY4742 transformed yeast expressing K28Alpha-gammaNS3, K28 pre-pro-Alpha subunit , the late under the control of galactose inducible promoter and the HCV NS3 isolate from the infected individual.

At the end of the incubation cell growth is measured, in each well, by absorbance at 600nm.

Cell growth accounts for HCV NS3 activity as the viral protease produces, through its action, the anti-toxin out of the pro-anti-toxin protein. Therefore HCV NS3 inhibition correlates to cell death measured as low absorbance at 600nm values.

The ability of the inhibiting compound to be active on the tested HCV NS3 isolate will depend on the inhibiting compound concentration that will induce 50% of cell death (or inhibit 50% of cell growth).

### 4) Method for determining a efficient therapeutic regimen

HCV NS3 protease was RT-PCR amplified out of purified total circulating RNA from blood plasma from HCV infected individuals with primers NS3Fwd2 (SEQ id n° 127) and NS3Rev1 (SEQ id n°128). The viral protease encoding DNA fragment was inserted in the cloning site of p415adh-NS4A expression vector. BY4742 yeast strain, harboring K28Alpha-gammaNS3 and K28 pre-pro-Alpha subunit, the late under the control of galactose inducible promoter, was further transformed with the plasmid carrying a unique HCV NS3 infecting variant isolate.

In 11 wells within a 96 well plate, 10 different concentrations (one well has no protease inhibitor) of either telaprevir or boceprevir, viral protease inhibiting compounds, were incubated for 48 hours at 30°C in 300µl of galactose containing liquid with 10⁵ of either BY4742 transformed yeast expressing K28Alpha-gammaNS3, K28 pre-pro-Alpha subunit, the late under the control of galactose inducible promoter, and the HCV NS3 and NS4A isolate from the infected individual, or BY4742 transformed yeast expressing K28Alpha-gammaNS3, K28 pre-pro-Alpha subunit, the late under the control of galactose inducible promoter, and a wild type protease inhibitor sensitive HCV NS3 and NS4A isolate.

At the end of the incubation cell growth is measured, in each well, by absorbance at 600nm.

Cell growth accounts for HCV NS3 activity as the viral protease produces, through its action, the anti-toxin out of the pro-anti-toxin protein. Therefore HCV NS3 inhibition correlates to cell death measured as low absorbance at 600nm values.

The ability of the inhibiting compound to be active on the tested HCV NS3 isolate will depend on the inhibiting compound concentration that will induce 50% of cell death (or inhibit 50% of cell growth), namely the IC₅₀ value.

The ratio of the IC₅₀ value for a certain inhibitor determined for a viral protease to be tested to the wild type sensitive protease defines the sensitive/resistant character of the tested isolate where an IC₅₀ ratio equal or lower than 1 implies sensitivity to highly sensitive to the specific compound thus becoming an indication for therapeutic regime choice.

## Claims

1. A vector coding for an antitoxin comprising a nucleic acid sequence under the control of a promoter, wherein:
- said nucleic acid sequence encodes a pro-antitoxin comprising the sequence of the cleavage site of a HCV protease,
- said pro-antitoxin has to be cleaved by said HCV protease to be activated.

2. The vector of claim 1, wherein said antitoxin is directed against a toxin corresponding to a cytotoxin, preferably against K28.

3. The vector of claim 2, wherein said antitoxin is directed against K28 and wherein the pro-antitoxin is a polypeptide, which N-terminal extremity comprises a derivative of the sequence SEQ id n°109, said derivative consisting in the insertion of a HCV cleavage site between the position 100 and 126 of SEQ id n° 109

4. The vector of claim 3, wherein the HCV protease is NS3-4A and the NS3-4A protease site is selected in the group comprising SEQ id n°4 to SEQ id n° 108.

5. The vector of claim 4, wherein the pro-anti-toxin is a polypeptide consisting in the sequence SEQ id n° 110.

6. A cell transformed with the vector of any one of claims 1 to 5.

7. The cell of claim 6, wherein:
- the vector is as defined in any one of claims 3 to 5, and
- Said cell is further transformed by a vector coding for the toxin K28 under the control of an inducible promoter.

8. The cell of any one of claim 6 or 7, wherein said cell is further transformed by a vector coding for the HCV protease activating the pro-antitoxin, preferably by a vector coding for the NS3-4A HCV protease.

9. The cell of any one of claims 6 to 8, wherein said cell a yeast cell, preferably *Saccharomyces cerevisiae.*

10. A method for screening a compound with the ability to inhibit HCV protease comprising the steps of:
i) contacting the cell as defined in any one of claims 6 to 9 with the compound,
ii) culturing the cell in a selective media so as to contact the cell with the toxin, and
iii) detecting inhibition of growth of the cell, a compound that inhibits growth of the cell being a compound that inhibits HCV protease.

11. The method of claim 10, wherein:
a) the toxin is K28, preferably SEQ id n°2, and the cell of step (i) was further transformed by a vector coding for K28 under the control of an inducible promoter;
b) the antitoxin is directed against K28, and the pro-antitoxin is a polypeptide, which N-terminal extremity comprises a derivative of the sequence SEQ id n°109, said derivative consisting in the insertion of a HCV cleavage site between the position 100 and 126 of SEQ id n° 109.

12. The method of any one of claims 10 or 11, wherein said method is for further determining the IC₅₀ of the compound for a HCV protease, and the successive steps i), ii) and iii) are repeated with different concentration of the compound.

13. The method of any one of claims 10 to 12, wherein said method is for further determining the ability of the compound to inhibit the HCV proteases expressed by different isolates of HCV viruses, and wherein the successive steps i), ii) and iii) are repeated with distinct transformed cells, each transformed cell expressing the HCV protease of one isolate of HCV virus.

14. The method of any one of claims 10 to 13, wherein said method is for further determining an efficient therapeutic regimen that can be used for treating a subject suffering from a HCV infection, wherein the successive steps i), ii) and iii) are repeated with each compound to be tested with:
a) said compound being selected in the group comprising inhibitors from a HCV protease, and
b) said transformed cell corresponding to a pool of transformed cells, which pool of cells has been transformed by:
1) a vector coding for an antitoxin comprising a nucleic acid sequence under the control of a promoter, wherein said nucleic acid sequence encodes a pro-antitoxin comprising the sequence of a cleavage site of the HCV protease, and said pro-antitoxin has to be cleaved by said HCV protease to be activated; and
2) a pool of vectors coding for the pool of HCV proteases expressed by the HCV viruses infecting said subject.

15. A kit for screening a compound with the ability to inhibit HCV protease, wherein said kit comprises:
i) a cell transformed by a vector coding for an antitoxin comprising a nucleic acid sequence under the control of a promoter, wherein said nucleic acid sequence encodes a pro-antitoxin comprising the sequence of a cleavage site of a HCV protease, and said pro-antitoxin has to be cleaved by said HCV protease to be activated
ii) a set of primers for amplifying a DNA fragment comprising at least one sequence coding for a HCV protease; and
iii) a vector enabling the cloning of said DNA fragment and the expression of said HCV protease in the cell after its transformation.


SEQUENCE LISTING

<110>  AMIKANA Biologics
       GLUSCHANKOF, Pablo
       Perrin-East, christèle

<120>  NEW METHOD FOR TESTING HCV PROTEASE INHIBITION

<130>  AMI-B-0005-EP1

<160>  130

<170>  PatentIn version 3.5

<210>  1
<211>  345
<212>  PRT
<213>  Artificial Sequence

<220>
<223>  M28 virus

<400>  1

Met Glu Ser Val Ser Ser Leu Phe Asn Ile Phe Ser Thr Ile Met Val
1               5                   10                  15

Asn Tyr Lys Ser Leu Val Leu Ala Leu Leu Ser Val Ser Asn Leu Lys
            20                  25                  30

Tyr Ala Arg Gly Met Pro Thr Ser Glu Arg Gln Gln Gly Leu Glu Glu
        35                  40                  45

Arg Asp Phe Ser Ala Ala Thr Cys Val Leu Met Gly Ala Glu Val Gly
    50                  55                  60

Ser Trp Gly Met Val Tyr Ser Gly Gln Lys Val Glu Ser Trp Ile Leu
65                  70                  75                  80

Tyr Val Leu Thr Gly Ile Thr Thr Met Ser Ala Ile Val Asp Glu Ile
                85                  90                  95

Asp Tyr Tyr Ala Ser His Met Pro Leu Ser Val Val Gly Glu Asn Ser
            100                 105                 110

Gly Leu Gln Ile Val Arg Asp Thr Ile Val Thr Leu Val Met Ala Gly
        115                 120                 125

Leu Thr Ala Ser Ala Asn Lys Val Ile Ser Lys Thr Glu Asn Ala Glu
    130                 135                 140

Asn Ile Gln Ser Arg Ser Leu Ile Pro Gly Leu Leu Ser Met Asp Tyr
145                 150                 155                 160

Asn Ser Thr His Thr Met Ala Ile Asn Leu Glu Asp Val Phe Ser Glu
                165                 170                 175

Leu Gly Trp Asp Ile Asp Thr Ser Asp Ser Ser Gly Leu Tyr Lys Arg
            180                 185                 190

Asp Asp Asn Ser Val Thr Leu His Leu Gly Asp Val Pro Ala Leu Gly
        195                 200                 205

Thr Ser Asn Thr Ile Ile Pro Asn Ala Val Met Gln Ile Tyr Asn Asn
    210                 215                 220

Ala Ser Phe Ala Phe Gly Phe Ala Pro His Ser Asn Gly Asn Ser Thr
225                 230                 235                 240

Gly Leu Gln Lys Arg Ala Ser Ile Asp Asp Ala Val Trp Leu Gln Ser
                245                 250                 255

Ala Tyr Gly Ile Ala Tyr Ser Ala Trp Ile Gly Ser Glu Asn Val Gly
            260                 265                 270

Ser Tyr Asp Gln His Leu Ala Glu Ala Asn Gly Met Ala Asn Tyr Trp
        275                 280                 285

Thr Ser Glu Cys Ser Lys Tyr Asn Gly Val Ile Trp Gly Asp Glu Ser
    290                 295                 300

Asp Ala Cys Gly Asn Trp Leu Ala Ser Gln Arg Leu Asp Ile Val Ser
305                 310                 315                 320

His Ser Thr Gly Asn Tyr Tyr Arg Asp Val Asn Leu Cys Gly Asp Asp
                325                 330                 335

Glu Ala Arg Cys His Asp Glu Leu Arg
            340                 345

<210>  2
<211>  149
<212>  PRT
<213>  Artificial Sequence

<220>
<223>  K28 pre-pro-alpha subunit

<400>  2

Met Glu Ser Val Ser Ser Leu Phe Asn Ile Phe Ser Thr Ile Met Val
1               5                   10                  15

Asn Tyr Lys Ser Leu Val Leu Ala Leu Leu Ser Val Ser Asn Leu Lys
            20                  25                  30

Tyr Ala Arg Gly Met Pro Thr Ser Glu Arg Gln Gln Gly Leu Glu Glu
        35                  40                  45

Arg Asp Phe Ser Ala Ala Thr Cys Val Leu Met Gly Ala Glu Val Gly
    50                  55                  60

Ser Trp Gly Met Val Tyr Ser Gly Gln Lys Val Glu Ser Trp Ile Leu
65                  70                  75                  80

Tyr Val Leu Thr Gly Ile Thr Thr Met Ser Ala Ile Val Asp Glu Ile
                85                  90                  95

Asp Tyr Tyr Ala Ser His Met Pro Leu Ser Val Val Gly Glu Asn Ser
            100                 105                 110

Gly Leu Gln Ile Val Arg Asp Thr Ile Val Thr Leu Val Met Ala Gly
        115                 120                 125

Leu Thr Ala Ser Ala Asn Lys Val Ile Ser Lys Thr Glu Asn Ala Glu
    130                 135                 140

Asn Ile Gln Ser Arg
145

<210>  3
<211>  101
<212>  PRT
<213>  Artificial Sequence

<220>
<223>  K28 alpha subuniot with antitoxin activity

<400>  3

Met Asp Phe Ser Ala Ala Thr Cys Val Leu Met Gly Ala Glu Val Gly
1               5                   10                  15

Ser Trp Gly Met Val Tyr Ser Gly Gln Lys Val Glu Ser Trp Ile Leu
            20                  25                  30

Tyr Val Leu Thr Gly Ile Thr Thr Met Ser Ala Ile Val Asp Glu Ile
        35                  40                  45

Asp Tyr Tyr Ala Ser His Met Pro Leu Ser Val Val Gly Glu Asn Ser
    50                  55                  60

Gly Leu Gln Ile Val Arg Asp Thr Ile Val Thr Leu Val Met Ala Gly

65                  70                  75                  80

Leu Thr Ala Ser Ala Asn Lys Val Ile Ser Lys Thr Glu Asn Ala Glu
                85                  90                  95

Asn Ile Gln Ser Arg
            100

<210>  4
<211>  11
<212>  PRT
<213>  Artificial Sequence

<220>
<223>  NS3 cleavage site

<400>  4

Cys Met Ala Ala Asp Leu Glu Val Ile Thr Ser
1               5                   10

<210>  5
<211>  11
<212>  PRT
<213>  Artificial Sequence

<220>
<223>  NS3 cleavage site

<400>  5

Cys Met Ser Ala Asp Leu Glu Val Ile Thr Ser
1               5                   10

<210>  6
<211>  11
<212>  PRT
<213>  Artificial Sequence

<220>
<223>  NS3 cleavage site

<400>  6

Cys Met Ala Ala Asp Leu Glu Val Val Thr Ser
1               5                   10

<210>  7
<211>  11
<212>  PRT
<213>  Artificial Sequence

<220>
<223>  NS3 cleavage site

<400>  7

Cys Met Ser Ala Asp Leu Glu Val Thr Thr Ser
1               5                   10


<210>  8
<211>  11
<212>  PRT
<213>  Artificial Sequence

<220>
<223>  NS3 cleavage site

<400>  8

Cys Met Ala Ala Asp Leu Glu Val Ala Thr Ser
1               5                   10


<210>  9
<211>  11
<212>  PRT
<213>  Artificial Sequence

<220>
<223>  NS3 cleavage site

<400>  9

Cys Met Gln Ala Asp Leu Glu Val Met Thr Ser
1               5                   10


<210>  10
<211>  11
<212>  PRT
<213>  Artificial Sequence

<220>
<223>  NS3 cleavage site

<400>  10

Cys Met Gln Ala Asp Leu Glu Ile Met Thr Ser
1               5                   10


<210>  11
<211>  11
<212>  PRT
<213>  Artificial Sequence

<220>
<223>  NS3 cleavage site

<400>  11

Cys Met Gln Ala Asp Leu Glu Val Met Thr Ser
1               5                   10


<210>  12
<211>  11
<212>  PRT

<213>  Artificial Sequence

<220>
<223>  NS3 cleavage site

<400>  12

Tyr Gln Glu Phe Asp Glu Met Glu Glu Cys Ser
1               5                   10


<210>  13
<211>  11
<212>  PRT
<213>  Artificial Sequence

<220>
<223>  NS3 cleavage site

<400>  13

Tyr Arg Glu Phe Asp Glu Met Glu Glu Cys Ser
1               5                   10


<210>  14
<211>  11
<212>  PRT
<213>  Artificial Sequence

<220>
<223>  NS3 cleavage site

<400>  14

Tyr Arg Glu Phe Asp Glu Met Glu Glu Cys Ala
1               5                   10


<210>  15
<211>  11
<212>  PRT
<213>  Artificial Sequence

<220>
<223>  NS3 cleavage site

<400>  15

Tyr Gln Glu Phe Asp Glu Met Glu Glu Cys Ala
1               5                   10


<210>  16
<211>  11
<212>  PRT
<213>  Artificial Sequence

<220>
<223>  NS3 cleavage site

<400>  16

Tyr Gln Gln Phe Asp Glu Met Glu Glu Cys Ser
1               5                   10

<210>  17
<211>  11
<212>  PRT
<213>  Artificial Sequence

<220>
<223>  NS3 cleavage site

<400>  17

Tyr Lys Gln Phe Asp Glu Met Glu Glu Cys Ser
1               5                   10

<210>  18
<211>  11
<212>  PRT
<213>  Artificial Sequence

<220>
<223>  NS3 cleavage site

<400>  18

Tyr Arg Gln Phe Asp Glu Met Glu Glu Cys Ser
1               5                   10

<210>  19
<211>  11
<212>  PRT
<213>  Artificial Sequence

<220>
<223>  NS3 cleavage site

<400>  19

Tyr Gln Gln Tyr Asp Glu Met Glu Glu Cys Ser
1               5                   10

<210>  20
<211>  11
<212>  PRT
<213>  Artificial Sequence

<220>
<223>  NS3 cleavage site

<400>  20

Phe Arg Gln Tyr Asp Glu Met Glu Glu Cys Ser
1               5                   10

<210>  21
<211>  11
<212>  PRT

<213>  Artificial Sequence

<220>
<223>  NS3 cleavage site

<400>  21

Tyr Gln Gln Phe Asp Glu Met Glu Glu Cys Ala
1               5                   10


<210>  22
<211>  11
<212>  PRT
<213>  Artificial Sequence

<220>
<223>  NS3 cleavage site

<400>  22

Tyr Glu Ala Phe Asp Glu Met Glu Glu Cys Ala
1               5                   10


<210>  23
<211>  11
<212>  PRT
<213>  Artificial Sequence

<220>
<223>  NS3 cleavage site

<400>  23

Trp Ile Ser Ser Glu Cys Thr Thr Pro Cys Ser
1               5                   10


<210>  24
<211>  11
<212>  PRT
<213>  Artificial Sequence

<220>
<223>  NS3 cleavage site

<400>  24

Trp Leu Ser Ser Glu Ser Thr Thr Pro Cys Ser
1               5                   10


<210>  25
<211>  11
<212>  PRT
<213>  Artificial Sequence

<220>
<223>  NS3 cleavage site

<400>  25

Trp Ile Ser Ser Asp Cys Thr Ala Pro Cys Ala
1               5                   10


<210>  26
<211>  11
<212>  PRT
<213>  Artificial Sequence

<220>
<223>  NS3 cleavage site

<400>  26

Trp Ile Ser Ser Asp Cys Ile Ala Pro Cys Ala
1               5                   10


<210>  27
<211>  11
<212>  PRT
<213>  Artificial Sequence

<220>
<223>  NS3 cleavage site

<400>  27

Trp Ile Asn Glu Asp Cys Ser Thr Pro Cys Ser
1               5                   10


<210>  28
<211>  11
<212>  PRT
<213>  Artificial Sequence

<220>
<223>  NS3 cleavage site

<400>  28

Trp Ile Asn Glu Asp Cys Thr Thr Pro Cys Ser
1               5                   10


<210>  29
<211>  11
<212>  PRT
<213>  Artificial Sequence

<220>
<223>  NS3 cleavage site

<400>  29

Trp Ile Asn Glu Asp Cys Ser Thr Pro Cys Ala
1               5                   10


<210>  30
<211>  11
<212>  PRT

<213>  Artificial Sequence

<220>
<223>  NS3 cleavage site

<400>  30

Trp Ile Asn Glu Asp Cys Pro Thr Pro Cys Asp
1               5                   10


<210>  31
<211>  11
<212>  PRT
<213>  Artificial Sequence

<220>
<223>  NS3 cleavage site

<400>  31

Trp Ile Asn Glu Asp His Ala Thr Pro Cys Thr
1               5                   10


<210>  32
<211>  11
<212>  PRT
<213>  Artificial Sequence

<220>
<223>  NS3 cleavage site

<400>  32

Trp Ile Asn Glu Asp Cys Ser Ala Pro Cys Asp
1               5                   10


<210>  33
<211>  11
<212>  PRT
<213>  Artificial Sequence

<220>
<223>  NS3 cleavage site

<400>  33

Trp Ile Gly Glu Asp Tyr Ser Thr Pro Cys Asp
1               5                   10


<210>  34
<211>  11
<212>  PRT
<213>  Artificial Sequence

<220>
<223>  NS3 cleavage site

<400>  34

Trp Ile Asn Gly Asp Trp Thr Thr Pro Cys Ala
1               5                   10


<210>  35
<211>  11
<212>  PRT
<213>  Artificial Sequence

<220>
<223>  NS3 cleavage site

<400>  35

Trp Ile Ser Gly Asp Trp Ser Ala Pro Cys Ser
1               5                   10


<210>  36
<211>  11
<212>  PRT
<213>  Artificial Sequence

<220>
<223>  NS3 cleavage site

<400>  36

Trp Ile Asn Thr Asp Trp Ser Thr Pro Cys Asn
1               5                   10


<210>  37
<211>  11
<212>  PRT
<213>  Artificial Sequence

<220>
<223>  NS3 cleavage site

<400>  37

Trp Ile Asn Ser Asp Trp Ser Ala Pro Cys Ser
1               5                   10


<210>  38
<211>  11
<212>  PRT
<213>  Artificial Sequence

<220>
<223>  NS3 cleavage site

<400>  38

Trp Ile Asn Gly Asp Trp Thr Thr Pro Cys Ser
1               5                   10


<210>  39
<211>  11
<212>  PRT

<213>  Artificial Sequence

<220>
<223>  NS3 cleavage site

<400>  39

Trp Ile Thr Glu Asp Tyr Ala Thr Pro Cys Gly
1               5                   10


<210>  40
<211>  11
<212>  PRT
<213>  Artificial Sequence

<220>
<223>  NS3 cleavage site

<400>  40

Trp Ile Asn Thr Asp Trp Ser Thr Pro Cys Ser
1               5                   10


<210>  41
<211>  11
<212>  PRT
<213>  Artificial Sequence

<220>
<223>  NS3 cleavage site

<400>  41

Trp Ile Asn Gly Asp Trp Ser Thr Pro Cys Ser
1               5                   10


<210>  42
<211>  11
<212>  PRT
<213>  Artificial Sequence

<220>
<223>  NS3 cleavage site

<400>  42

Trp Ile Asn Gly Asp Trp Ser Thr Pro Cys Ala
1               5                   10


<210>  43
<211>  11
<212>  PRT
<213>  Artificial Sequence

<220>
<223>  NS3 cleavage site

<400>  43

Trp Ile Asn Ser Asp Trp Ser Thr Pro Cys Ser
1               5                   10


<210>  44
<211>  11
<212>  PRT
<213>  Artificial Sequence

<220>
<223>  NS3 cleavage site

<400>  44

Trp Val Asn Ser Asp Trp Ser Thr Pro Cys Ala
1               5                   10


<210>  45
<211>  11
<212>  PRT
<213>  Artificial Sequence

<220>
<223>  NS3 cleavage site

<400>  45

Trp Ile Asn Thr Asp Trp Thr Thr Pro Cys Ser
1               5                   10


<210>  46
<211>  11
<212>  PRT
<213>  Artificial Sequence

<220>
<223>  NS3 cleavage site


<220>
<221>  misc_feature
<222>  (7)..(7)
<223>  Xaa can be any naturally occurring amino acid

<400>  46

Trp Ile Asn Thr Asp Trp Xaa Thr Pro Cys Ser
1               5                   10


<210>  47
<211>  11
<212>  PRT
<213>  Artificial Sequence

<220>
<223>  NS3 cleavage site

<400>  47

Trp Ile Asn Glu Asp Trp Ser Thr Pro Cys Ser

1               5                   10


<210>  48
<211>  11
<212>  PRT
<213>  Artificial Sequence

<220>
<223>  NS3 cleavage site

<400>  48

Trp Val Asn Glu Asp Thr Ala Thr Pro Cys Ala
1               5                   10


<210>  49
<211>  11
<212>  PRT
<213>  Artificial Sequence

<220>
<223>  NS3 cleavage site

<400>  49

Trp Ile His Glu Asp Thr Ser Thr Pro Cys Ala
1               5                   10


<210>  50
<211>  11
<212>  PRT
<213>  Artificial Sequence

<220>
<223>  NS3 cleavage site

<400>  50

Trp Ile Asn Glu Asp Tyr Pro Ser Pro Cys Ser
1               5                   10


<210>  51
<211>  11
<212>  PRT
<213>  Artificial Sequence

<220>
<223>  NS3 cleavage site

<400>  51

Trp Ile Asn Glu Asp Tyr Pro Ser Pro Cys Asn
1               5                   10


<210>  52
<211>  11
<212>  PRT
<213>  Artificial Sequence

<220>
<223>  NS3 cleavage site

<400>  52

Trp Ile Asn Glu Asp Tyr Pro Ser Pro Cys Ala
1               5                   10


<210>  53
<211>  11
<212>  PRT
<213>  Artificial Sequence

<220>
<223>  NS3 cleavage site

<400>  53

Trp Ile Asn Asp Asp Tyr Gln Ser Pro Cys Ser
1               5                   10


<210>  54
<211>  11
<212>  PRT
<213>  Artificial Sequence

<220>
<223>  NS3 cleavage site

<400>  54

Trp Ile Asn Glu Asp Tyr Pro Thr Pro Cys Asp
1               5                   10


<210>  55
<211>  11
<212>  PRT
<213>  Artificial Sequence

<220>
<223>  NS3 cleavage site

<400>  55

Trp Ile Thr Glu Asp Cys Pro Ile Pro Cys Ser
1               5                   10


<210>  56
<211>  11
<212>  PRT
<213>  Artificial Sequence

<220>
<223>  NS3 cleavage site

<400>  56

Trp Ile Thr Glu Asp Cys Pro Val Pro Cys Ser

1                5                   10


<210>  57
<211>  11
<212>  PRT
<213>  Artificial Sequence

<220>
<223>  NS3 cleavage site

<400>  57

Trp Ile Ser Glu Asp Tyr Pro Val Pro Cys Ala
1               5                   10


<210>  58
<211>  11
<212>  PRT
<213>  Artificial Sequence

<220>
<223>  NS3 cleavage site

<400>  58

Trp Ile Thr Glu Asp Cys Pro Val Pro Cys Ala
1               5                   10


<210>  59
<211>  11
<212>  PRT
<213>  Artificial Sequence

<220>
<223>  NS3 cleavage site

<400>  59

Trp Ile Thr Asp Asp Cys Pro Ile Pro Cys Ala
1               5                   10


<210>  60
<211>  11
<212>  PRT
<213>  Artificial Sequence

<220>
<223>  NS3 cleavage site

<400>  60

Trp Ile Thr Asp Asp Cys Pro Val Pro Cys Ala
1               5                   10


<210>  61
<211>  11
<212>  PRT
<213>  Artificial Sequence

<220>
<223>  NS3 cleavage site

<400>  61

Trp Ile Thr Glu Asp Cys Pro Val Pro Cys Ser
1               5                   10


<210>  62
<211>  11
<212>  PRT
<213>  Artificial Sequence

<220>
<223>  NS3 cleavage site

<400>  62

Gly Ala Asp Thr Glu Asp Val Val Cys Cys Ser
1               5                   10


<210>  63
<211>  11
<212>  PRT
<213>  Artificial Sequence

<220>
<223>  NS3 cleavage site

<400>  63

Glu Ala Asn Ala Glu Asp Val Val Cys Cys Ser
1               5                   10


<210>  64
<211>  11
<212>  PRT
<213>  Artificial Sequence

<220>
<223>  NS3 cleavage site

<400>  64

Glu Ala Gly Thr Glu Asp Val Val Cys Cys Ser
1               5                   10


<210>  65
<211>  11
<212>  PRT
<213>  Artificial Sequence

<220>
<223>  NS3 cleavage site

<400>  65

Asp Gly Gly Thr Glu Asp Val Val Cys Cys Ser

1               5                   10


<210>  66
<211>  11
<212>  PRT
<213>  Artificial Sequence

<220>
<223>  NS3 cleavage site

<400>  66

Glu Glu Ala Ser Glu Asp Val Val Cys Cys Ser
1               5                   10


<210>  67
<211>  11
<212>  PRT
<213>  Artificial Sequence

<220>
<223>  NS3 cleavage site

<400>  67

Gly Glu Ala Ser Asp Asp Ile Val Cys Cys Ser
1               5                   10


<210>  68
<211>  11
<212>  PRT
<213>  Artificial Sequence

<220>
<223>  NS3 cleavage site

<400>  68

Glu Glu Ala Gly Glu Asp Val Ile Cys Cys Ser
1               5                   10


<210>  69
<211>  11
<212>  PRT
<213>  Artificial Sequence

<220>
<223>  NS3 cleavage site

<400>  69

Gly Glu Ala Gly Glu Asp Val Val Cys Cys Ser
1               5                   10


<210>  70
<211>  11
<212>  PRT
<213>  Artificial Sequence

<220>
<223>  NS3 cleavage site

<400>  70

Glu Glu Asp Gly Glu Gly Val Ile Cys Cys Ser
1               5                   10


<210>  71
<211>  11
<212>  PRT
<213>  Artificial Sequence

<220>
<223>  NS3 cleavage site

<400>  71

Val Ser Gly Thr Glu Asp Val Val Cys Cys Ser
1               5                   10


<210>  72
<211>  11
<212>  PRT
<213>  Artificial Sequence

<220>
<223>  NS3 cleavage site

<400>  72

Val Ser Gly Ser Glu Asp Val Val Cys Cys Ser
1               5                   10


<210>  73
<211>  11
<212>  PRT
<213>  Artificial Sequence

<220>
<223>  NS3 cleavage site

<400>  73

Val Ser Glu Ser Glu Asp Val Val Cys Cys Ser
1               5                   10


<210>  74
<211>  11
<212>  PRT
<213>  Artificial Sequence

<220>
<223>  NS3 cleavage site

<400>  74

Val Ser Glu Thr Glu Asp Val Val Cys Cys Ser

1               5                   10


<210>  75
<211>  11
<212>  PRT
<213>  Artificial Sequence

<220>
<223>  NS3 cleavage site

<400>  75

Val Ser Ser Ser Glu Asp Val Val Cys Cys Ser
1               5                   10


<210>  76
<211>  11
<212>  PRT
<213>  Artificial Sequence

<220>
<223>  NS3 cleavage site

<400>  76

Val Ser Thr Asn Glu Asp Val Val Cys Cys Ser
1               5                   10


<210>  77
<211>  11
<212>  PRT
<213>  Artificial Sequence

<220>
<223>  NS3 cleavage site

<400>  77

Val Ser Ser Ser Asp Asp Val Val Cys Cys Ser
1               5                   10


<210>  78
<211>  11
<212>  PRT
<213>  Artificial Sequence

<220>
<223>  NS3 cleavage site

<400>  78

Val Ser Gly Glu Asp Asn Val Val Cys Cys Ser
1               5                   10


<210>  79
<211>  11
<212>  PRT
<213>  Artificial Sequence

<220>
<223>  NS3 cleavage site

<400>  79

Val Ser Asp Glu Asp Ser Val Val Cys Cys Ser
1               5                   10


<210>  80
<211>  11
<212>  PRT
<213>  Artificial Sequence

<220>
<223>  NS3 cleavage site

<400>  80

Asp Asp Ala Asn Ser Ser Val Val Cys Cys Ser
1               5                   10


<210>  81
<211>  11
<212>  PRT
<213>  Artificial Sequence

<220>
<223>  NS3 cleavage site

<400>  81

Ser Glu Asp His Asp Ser Val Val Cys Cys Ser
1               5                   10


<210>  82
<211>  11
<212>  PRT
<213>  Artificial Sequence

<220>
<223>  NS3 cleavage site

<400>  82

Ser Glu Glu Gly Asp Ser Val Val Cys Cys Ser
1               5                   10


<210>  83
<211>  11
<212>  PRT
<213>  Artificial Sequence

<220>
<223>  NS3 cleavage site

<400>  83

Glu Ala Glu Asn Asp Ser Val Val Cys Cys Ser

1                   5                   10


<210>  84
<211>  11
<212>  PRT
<213>  Artificial Sequence

<220>
<223>  NS3 cleavage site

<400>  84

Val Ser Glu Glu Thr Ser Val Val Cys Cys Ser
1               5                   10


<210>  85
<211>  11
<212>  PRT
<213>  Artificial Sequence

<220>
<223>  NS3 cleavage site

<400>  85

Ser Glu Asp Gly Asp Ser Val Val Cys Cys Ser
1               5                   10


<210>  86
<211>  11
<212>  PRT
<213>  Artificial Sequence

<220>
<223>  NS3 cleavage site

<400>  86

Ser Ser Glu Glu Thr Ser Val Val Cys Cys Ser
1               5                   10


<210>  87
<211>  11
<212>  PRT
<213>  Artificial Sequence

<220>
<223>  NS3 cleavage site

<400>  87

Ser Glu Glu Gly Asp Ser Val Ile Cys Cys Ser
1               5                   10


<210>  88
<211>  11
<212>  PRT
<213>  Artificial Sequence

<220>
<223>  NS3 cleavage site

<400>  88

Ser Glu Glu Gly Asp Gly Val Val Cys Cys Ser
1               5                   10


<210>  89
<211>  11
<212>  PRT
<213>  Artificial Sequence

<220>
<223>  NS3 cleavage site

<400>  89

Ser Asp Glu Asp Glu Ser Met Ile Cys Cys Ser
1               5                   10


<210>  90
<211>  11
<212>  PRT
<213>  Artificial Sequence

<220>
<223>  NS3 cleavage site

<400>  90

Ser Glu Glu Gly Asp Ser Val Val Cys Cys Ser
1               5                   10


<210>  91
<211>  11
<212>  PRT
<213>  Artificial Sequence

<220>
<223>  NS3 cleavage site

<400>  91

Ser Glu Glu Glu Asp Ser Ile Ile Cys Cys Ser
1               5                   10


<210>  92
<211>  11
<212>  PRT
<213>  Artificial Sequence

<220>
<223>  NS3 cleavage site

<400>  92

Val Ser Glu Glu Glu Ser Val Val Cys Cys Ser

1               5                   10


<210>  93
<211>  11
<212>  PRT
<213>  Artificial Sequence

<220>
<223>  NS3 cleavage site

<400>  93

Val Ser Asp Gln Asp Asp Val Val Cys Cys Ser
1               5                   10


<210>  94
<211>  11
<212>  PRT
<213>  Artificial Sequence

<220>
<223>  NS3 cleavage site

<400>  94

Val Ser Asp Glu Asp Asp Val Val Cys Cys Ser
1               5                   10


<210>  95
<211>  11
<212>  PRT
<213>  Artificial Sequence

<220>
<223>  NS3 cleavage site

<400>  95

Val Ser Glu Glu Asp Asp Val Val Cys Cys Ser
1               5                   10


<210>  96
<211>  11
<212>  PRT
<213>  Artificial Sequence

<220>
<223>  NS3 cleavage site

<400>  96

Asp Asn Glu Glu Gln Ser Val Val Cys Cys Ser
1               5                   10


<210>  97
<211>  11
<212>  PRT
<213>  Artificial Sequence

<220>
<223>  NS3 cleavage site

<400>  97

Asp Ser Glu Glu Gln Ser Val Ile Cys Cys Ser
1               5                   10


<210>  98
<211>  11
<212>  PRT
<213>  Artificial Sequence

<220>
<223>  NS3 cleavage site

<400>  98

Asp Ser Glu Glu Gln Ser Val Val Cys Cys Ser
1               5                   10


<210>  99
<211>  11
<212>  PRT
<213>  Artificial Sequence

<220>
<223>  NS3 cleavage site

<400>  99

Ser Gly Glu Glu Gln Ser Val Val Cys Cys Ser
1               5                   10


<210>  100
<211>  11
<212>  PRT
<213>  Artificial Sequence

<220>
<223>  NS3 cleavage site

<400>  100

Ser Glu Glu Asp Asp Ser Val Val Cys Cys Ser
1               5                   10


<210>  101
<211>  11
<212>  PRT
<213>  Artificial Sequence

<220>
<223>  NS3 cleavage site

<400>  101

Ser Glu Glu Asp Asp Thr Thr Val Cys Cys Ser

1               5                   10


<210>  102
<211>  11
<212>  PRT
<213>  Artificial Sequence

<220>
<223>  NS3 cleavage site

<400>  102

Ser Asp Val Asp Asp Ser Val Val Cys Cys Ser
1               5                   10


<210>  103
<211>  11
<212>  PRT
<213>  Artificial Sequence

<220>
<223>  NS3 cleavage site

<400>  103

Ser Asp Glu Gly Asp Ser Val Ile Cys Cys Ser
1               5                   10


<210>  104
<211>  11
<212>  PRT
<213>  Artificial Sequence

<220>
<223>  NS3 cleavage site

<400>  104

Ser Glu Glu Gly Gly Ser Glu Val Cys Cys Ser
1               5                   10


<210>  105
<211>  11
<212>  PRT
<213>  Artificial Sequence

<220>
<223>  NS3 cleavage site

<400>  105

Ser Asp Glu Asp Asp Ser Val Val Cys Cys Ser
1               5                   10


<210>  106
<211>  11
<212>  PRT
<213>  Artificial Sequence

<220>
<223>  NS3 cleavage site

<400>  106

Ser Asp Glu Gly Asp Ser Val Val Cys Cys Ser
1               5                   10


<210>  107
<211>  11
<212>  PRT
<213>  Artificial Sequence

<220>
<223>  NS3 cleavage site

<400>  107

Ser Asp Gln Glu Asp Ser Val Ile Cys Cys Ser
1               5                   10


<210>  108
<211>  11
<212>  PRT
<213>  Artificial Sequence

<220>
<223>  NS3 cleavage site

<400>  108

Cys Met Ser Ala Asp Leu Glu Val Val Thr Ser
1               5                   10


<210>  109
<211>  126
<212>  PRT
<213>  Artificial Sequence

<220>
<223>  K28 antitoxin

<400>  109

Met Asp Phe Ser Ala Ala Thr Cys Val Leu Met Gly Ala Glu Val Gly
1               5                   10                  15


Ser Trp Gly Met Val Tyr Ser Gly Gln Lys Val Glu Ser Trp Ile Leu
            20                  25                  30


Tyr Val Leu Thr Gly Ile Thr Thr Met Ser Ala Ile Val Asp Glu Ile
        35                  40                  45


Asp Tyr Tyr Ala Ser His Met Pro Leu Ser Val Val Gly Glu Asn Ser
    50                  55                  60

Gly Leu Gln Ile Val Arg Asp Thr Ile Val Thr Leu Val Met Ala Gly
65                  70                  75                  80

Leu Thr Ala Ser Ala Asn Lys Val Ile Ser Lys Thr Glu Asn Ala Glu
                85                  90                  95

Asn Ile Gln Ser Arg Ser Leu Ile Pro Gly Leu Leu Ser Met Asp Tyr
            100                 105                 110

Asn Ser Thr His Thr Met Ala Ile Asn Leu Glu Asp Val Phe
        115                 120                 125

<210>  110
<211>  197
<212>  PRT
<213>  Artificial Sequence

<220>
<223>  K28 antitoxin

<400>  110

Met Asp Phe Ser Ala Ala Thr Cys Val Leu Met Gly Ala Glu Val Gly
1               5                   10                  15

Ser Trp Gly Met Val Tyr Ser Gly Gln Lys Val Glu Ser Trp Ile Leu
            20                  25                  30

Tyr Val Leu Thr Gly Ile Thr Thr Met Ser Ala Ile Val Asp Glu Ile
        35                  40                  45

Asp Tyr Tyr Ala Ser His Met Pro Leu Ser Val Val Gly Glu Asn Ser
    50                  55                  60

Gly Leu Gln Ile Val Arg Asp Thr Ile Val Thr Leu Val Met Ala Gly
65                  70                  75                  80

Leu Thr Ala Ser Ala Asn Lys Val Ile Ser Lys Thr Glu Asn Ala Glu
                85                  90                  95

Asn Ile Gln Ser Arg Ser Leu Ile Pro Gly Leu Leu Ser Met Asp Tyr
            100                 105                 110

Asn Ser Thr His Thr Met Ala Ala Asp Leu Glu Val Val Thr Ser Glu
        115                 120                 125

Leu Gly Trp Asp Ile Asp Thr Ser Asp Ser Ser Gly Leu Tyr Lys Arg
    130                 135                 140

Asp Asp Asn Ser Val Thr Leu His Leu Gly Asp Val Pro Ala Leu Gly
145                 150                 155                 160

Thr Ser Asn Thr Ile Ile Pro Asn Ala Val Met Gln Ile Tyr Asn Asn
                165                 170                 175

Ala Ser Phe Ala Phe Gly Phe Ala Pro His Ser Asn Gly Asn Ser Thr
            180                 185                 190

Gly Leu Gln Lys Arg
        195

<210>  111
<211>  366
<212>  DNA
<213>  Artificial Sequence

<220>
<223>  GAL1 promoter

<400>  111
gatcaaaaat catcgcttcg ctgattaatt accccagaaa taaggctaaa aaactaatcg      60

cattatcatc ctatggttgt taatttgatt cgttcatttg aaggtttgtg gggccaggtt     120

actgccaatt tttcctcttc ataaccataa aagctagtat tgtagaatct ttattgttcg     180

gagcagtgcg gcgcgaggca catctgcgtt tcaggaacgc gaccggtgaa gacgaggacg     240

cacggaggag agtcttcctt cggagggctg tcacccgctc ggcggcttct aatccgtact     300

tcaatatagc aatgagcagt taagcgtatt actgaaagtt ccaaagagaa ggttttttta     360

ggctaa                                                                366

<210>  112
<211>  690
<212>  DNA
<213>  Artificial Sequence

<220>
<223>  ADH1 promoter

<400>  112
tgtacaatat ggacttcctc ttttctggca accaaaccca tacatcggga ttcctataat      60

accttcgttg gtctccctaa catgtaggtg gcggagggga gatatacaat agaacagata     120

ccagacaaga cataatgggc taaacaagac tacaccaatt acactgcctc attgatggtg     180

gtacataacg aactaatact gtagccctag acttgatagc catcatcata tcgaagtttc     240

actacccttt ttccatttgc catctattga agtaataata ggcgcatgca acttcttttc     300

tttttttttc ttttctctct cccccgttgt tgtctcacca tatccgcaat gacaaaaaaa     360

atgatggaag acactaaagg aaaaaattaa cgacaaagac agcaccaaca gatgtcgttg     420

ttccagagct gatgaggggt atcttcgaac acacgaaact ttttccttcc ttcattcacg 480

cacactactc tctaatgagc aacggtatac ggccttcctt ccagttactt gaatttgaaa 540

taaaaaaagt ttgccgcttt gctatcaagt ataaatagac ctgcaattat taatcttttg 600

tttcctcgtc attgttctcg ttccctttct tccttgtttc tttttctgca caatatttca 660

agctatacca agcatacaat caactatctc 690

<210> 113
<211> 655
<212> DNA
<213> Artificial Sequence

<220>
<223> G3PDH promoter

<400> 113
   agtttatcat tatcaatact gccatttcaa agaatacgta aataattaat agtagtgatt 60
   ttcctaactt tatttagtca aaaaattagc cttttaattc tgctgtaacc cgtacatgcc 120
   caaaataggg ggcgggttac acagaatata taacatcgta ggtgtctggg tgaacagttt 180
   attcctggca tccactaaat ataatggagc ccgcttttta agctggcatc cagaaaaaaa 240
   aagaatccca gcaccaaaat attgttttct tcaccaacca tcagttcata ggtccattct 300
   cttagcgcaa ctacagagaa caggggcaca aacaggcaaa aaacgggcac aacctcaatg 360
   gagtgatgca acctgcctgg agtaaatgat gacacaaggc aattgaccca cgcatgtatc 420
   tatctcattt tcttacacct tctattacct tctgctctct ctgatttgga aaaagctgaa 480
   aaaaaaggtt gaaaccagtt ccctgaaatt attcccctac ttgactaata agtatataaa 540
   gacggtaggt attgattgta attctgtaaa tctatttctt aaacttctta aattctactt 600
   ttatagttag tctttttttt agttttaaaa caccaagaac ttagtttcga cggat 655

<210> 114
<211> 624
<212> DNA
<213> Artificial Sequence

<220>
<223> teTR promoter

<400> 114
   atgtctagat tagataaaag taaagtgatt aacagcgcat tagagctgct taatgaggtc 60
   ggaatcgaag gtttaacaac ccgtaaactc gcccagaagc taggtgtaga gcagcctaca 120
   ttgtattggc atgtaaaaaa taagcgggct ttgctcgacg ccttagccat tgagatgtta 180
   gataggcacc atactcactt ttgcccttta gaaggggaaa gctggcaaga ttttttacgt 240
   aataacgcta aaagttttag atgtgcttta ctaagtcatc gcgatggagc aaaagtacat 300
   ttaggtacac ggcctacaga aaaacagtat gaaactctcg aaaatcaatt agccttttta 360
   tgccaacaag gtttttcact agagaatgca ttatatgcac tcagcgctgt ggggcatttt 420
   actttaggtt gcgtattgga agatcaagag catcaagtcg ctaaagaaga aagggaaaca 480
   cctactactg atagtatgcc gccattatta cgacaagcta tcgaattatt tgatcaccaa 540
   ggtgcagagc cagccttctt attcggcctt gaattgatca tatgcggatt agaaaaacaa 600
   cttaaatgtg aaagtggatc ctag 624

<210> 115
<211> 686
<212> PRT
<213> Hepatitis C virus

<400> 115

Met Ala Pro Ile Thr Ala Tyr Thr Gln Gln Thr Arg Gly Leu Leu Gly
1               5                   10                  15

Cys Ile Ile Thr Ser Leu Thr Gly Arg Asp Lys Asn Gln Val Glu Gly
            20                  25                  30

Glu Val Gln Ile Val Ser Thr Ala Thr Gln Thr Phe Leu Ala Thr Cys
        35                  40                  45

Ile Asn Gly Val Cys Trp Thr Val Tyr His Gly Ala Gly Thr Arg Thr
    50                  55                  60

Ile Ala Ser Pro Lys Gly Pro Val Ile Gln Met Tyr Thr Asn Val Asp
65                  70                  75                  80

Gln Asp Leu Val Gly Trp Pro Ala Pro Gln Gly Ser Arg Ser Leu Ala
                85                  90                  95

Pro Cys Thr Cys Gly Ser Ser Asp Leu Tyr Leu Val Thr Arg His Ala
            100                 105                 110

Asp Val Ile Pro Val Arg Arg Arg Gly Asp Ser Arg Gly Ser Leu Leu
        115                 120                 125

Ser Pro Arg Pro Ile Ser Tyr Leu Lys Gly Ser Ser Gly Gly Pro Leu
    130                 135                 140

Leu Cys Pro Ala Gly His Ala Val Gly Leu Phe Arg Ala Ala Val Cys
145                 150                 155                 160

Thr Arg Gly Val Ala Lys Ala Val Asp Phe Ile Pro Val Glu Asn Leu
                165                 170                 175

Gly Thr Thr Met Arg Ser Pro Val Phe Thr Asp Asn Ser Ser Pro Pro
            180                 185                 190

Ala Val Pro Gln Ser Phe Gln Val Ala His Leu His Ala Pro Thr Gly
        195                 200                 205

Ser Gly Lys Ser Thr Lys Val Pro Ala Ala Tyr Ala Ala Gln Gly Tyr
    210                 215                 220

Lys Val Leu Val Leu Asn Pro Ser Val Ala Ala Thr Leu Gly Phe Gly
225                 230                 235                 240

Ala Tyr Met Ser Lys Ala His Gly Val Asp Pro Asn Ile Arg Thr Gly
                245                 250                 255

Val Arg Thr Ile Thr Thr Gly Ser Pro Ile Thr Tyr Ser Thr Tyr Gly
            260                 265                 270

Lys Phe Leu Ala Asp Gly Gly Cys Ser Gly Gly Ala Tyr Asp Ile Ile
        275                 280                 285

Ile Cys Asp Glu Cys His Ser Thr Asp Ala Thr Ser Ile Leu Gly Ile
    290                 295                 300

Gly Thr Val Leu Asp Gln Ala Glu Thr Ala Gly Ala Arg Leu Val Val
305                 310                 315                 320

Leu Ala Thr Ala Thr Pro Pro Gly Ser Val Thr Val Ser His Pro Asn
                325                 330                 335

Ile Glu Glu Val Ala Leu Ser Thr Thr Gly Glu Ile Pro Phe Tyr Gly
            340                 345                 350

Lys Ala Ile Pro Leu Glu Val Ile Lys Gly Gly Arg His Leu Ile Phe
        355                 360                 365

Cys His Ser Lys Lys Lys Cys Asp Glu Leu Ala Ala Lys Leu Val Ala
    370                 375                 380

Leu Gly Ile Asn Ala Val Ala Tyr Tyr Arg Gly Leu Asp Val Ser Val
385                 390                 395                 400

Ile Pro Thr Ser Gly Asp Val Val Val Ser Thr Asp Ala Leu Met
                405                 410                 415

Thr Gly Phe Thr Gly Asp Phe Asp Ser Val Ile Asp Cys Asn Thr Cys
            420                 425                 430

Val Thr Gln Thr Val Asp Phe Ser Leu Asp Pro Thr Phe Thr Ile Glu
        435                 440                 445

Thr Thr Thr Leu Pro Gln Asp Ala Val Ser Arg Thr Gln Arg Arg Gly
    450                 455                 460

Arg Thr Gly Arg Gly Lys Pro Gly Ile Tyr Arg Phe Val Ala Pro Gly
465                 470                 475                 480

Glu Arg Pro Ser Gly Met Phe Asp Ser Ser Val Leu Cys Glu Cys Tyr
                485                 490                 495

Asp Ala Gly Cys Ala Trp Tyr Glu Leu Thr Pro Ala Glu Thr Thr Val
            500                 505                 510

Arg Leu Arg Ala Tyr Met Asn Thr Pro Gly Leu Pro Val Cys Gln Asp
        515                 520                 525

His Leu Glu Phe Trp Glu Gly Val Phe Thr Gly Leu Thr His Ile Asp
    530                 535                 540

Ala His Phe Leu Ser Gln Thr Lys Gln Ser Gly Glu Asn Phe Pro Tyr
545                 550                 555                 560

Leu Val Ala Tyr Gln Ala Thr Val Cys Ala Arg Ala Gln Ala Pro Pro
                565                 570                 575

Pro Ser Trp Asp Gln Met Trp Lys Cys Leu Ile Arg Leu Lys Pro Thr
            580                 585                 590

Leu His Gly Pro Thr Pro Leu Leu Tyr Arg Leu Gly Ala Val Gln Asn
        595                 600                 605

Glu Val Thr Leu Thr His Pro Ile Thr Lys Tyr Ile Met Thr Cys Met
    610                 615                 620

Ser Ala Asp Leu Glu Val Val Thr Ser Thr Trp Val Leu Val Gly Gly
625                 630                 635                 640

Val Leu Ala Ala Leu Ala Ala Tyr Cys Leu Ser Thr Gly Cys Val Val
                645                 650                 655

Ile Val Gly Arg Ile Val Leu Ser Gly Lys Pro Ala Ile Ile Pro Asp
            660                 665                 670

Arg Glu Val Leu Tyr Gln Glu Phe Asp Glu Met Glu Glu Cys
        675                 680                 685

<210>  116
<211>  39
<212>  DNA
<213>  Artificial Sequence

<220>
<223>  5'primer K28A

<400>  116
gaatgtgggt ctagaatgga cttcagtgct gctacttgc                              39


<210>  117
<211>  42
<212>  DNA
<213>  Artificial Sequence

<220>
<223>   5'primer K28ppA

<400>  117
tctagactcg aggaattcat ggagagcgtt tcctcattat tt                          42


<210>  118
<211>  30
<212>  DNA
<213>  Artificial Sequence

<220>
<223>  3'primerK28sac

<400>  118
gagctcttaa cgcgattgta tattctctgc                                        30


<210>  119
<211>  39
<212>  DNA
<213>  Artificial Sequence

<220>
<223>  5'alpha

<400>  119
gaatgtgggt ctagaatgga cttcagtgct gctacttgc                              39


<210>  120
<211>  27
<212>  DNA
<213>  Artificial Sequence

<220>
<223>  3'M28-100

<400>  120
ttaagctcgt ttctgcaagc ctgtaga                                           27


<210>  121

<211>  31
<212>  DNA
<213>  Artificial Sequence

<220>
<223>  3'M28-170

<400>  121
gctcttagaa tacgtcttcc aaattaatcg c                                    31


<210>  122
<211>  42
<212>  DNA
<213>  Artificial Sequence

<220>
<223>  5'beta

<400>  122
gaatgtgggt ctagaatgca gcatctagct gaagctaacg gt                        42


<210>  123
<211>  34
<212>  DNA
<213>  Artificial Sequence

<220>
<223>  3'M28

<400>  123
gtctggacta ttagcgtagc tcatcgtggc acct                                 34


<210>  124
<211>  36
<212>  DNA
<213>  Artificial Sequence

<220>
<223>  K28Alpha-gammaNS3 5'primer

<400>  124
acccatacca tggcggcgga tctggaagtg gtgacc                               36


<210>  125
<211>  36
<212>  DNA
<213>  Artificial Sequence

<220>
<223>  K28Alpha-gammaNS3 3'primer

<400>  125
ggtcaccact tccagatccg ccgccatggt atgggt                               36


<210>  126
<211>  39
<212>  DNA
<213>  Artificial Sequence

<220>
<223>  3'M28-170ns3

<400>  126
ttaggtcacc acttccagat ccgccgccat ggtatgggt                              39


<210>  127
<211>  20
<212>  DNA
<213>  Artificial Sequence

<220>
<223>  NS3Fwd2

<400>  127
atcacstggg grgcrgayac                                                   20


<210>  128
<211>  23
<212>  DNA
<213>  Artificial Sequence

<220>
<223>  NS3Rev1:

<400>  128
aayttgccrt akgtggagta ygt                                               23


<210>  129
<211>  20
<212>  DNA
<213>  Artificial Sequence

<220>
<223>  NS3 Fwd3

<400>  129
acsgcrgcrt gyggggacat                                                   20


<210>  130
<211>  20
<212>  DNA
<213>  Artificial Sequence

<220>
<223>  NS3Rev2

<400>  130
gtgctcttrc cgctrccrgt                                                   20